# EUROPEAN PATENT SPECIFICATION

(11) **EP 1 948 216 B1**
(45) Date of publication and mention of the grant of the patent: **30.03.2011**
(21) Application number: 06825898.7
(22) Date of filing: 12.10.2006
(51) Int. Cl.: A61K 38/17, A61P 13/12

(54) **TREATMENT OF ACUTE RENAL FAILURE WITH SOLUBLE THROMBOMODULIN**
BEHANDLUNG VON AKUTEM NIERENVERSAGEN MIT LÖSLICHEM THROMBOMODULIN
TRAITEMENT D'UNE INSUFFISANCE RENALE AIGUE AVEC LA THROMBOMODULINE SOLUBLE

(30) Priority: 13.10.2005 US 726376 P
(43) Date of publication of application: 30.07.2008
(73) Proprietor: ELI LILLY AND COMPANY, Indianapolis, IN 46285 (US); Indiana University Research and Technology Corporation, Indianapolis, IN 46202 (US)
(72) Inventor: GRINNELL, Brian, William, Indianapolis, Indiana 46220 (US); MCKINNEY, Thurman, Dwight, Deep Gap, North Carolina 28618 (US); MOLITORIS, Bruce, A., Indianapolis, Indiana 46260 (US)
(74) Representative: Commander, Paul Martin Brial
(86) International application number: PCT/US2006/040069
(87) International publication number: WO 2007/047430

(56) References cited:
- WO-A2-2004/076635
- IKEGUCHI HIROSHI ET AL: "Effects of recombinant human soluble thrombomodulin (RHS-TM) in experimental glomerular thrombosis in rats" JOURNAL OF THE AMERICAN SOCIETY OF NEPHROLOGY, vol. 10, no. PROGRAM AND ABSTR. ISSUE, September 1999 (1999-09), page 548A, XP009078182 & 32ND ANNUAL MEETING OF THE AMERICAN SOCIETY OF NEPHROLOGY; MIAMI BEACH, FLORIDA, USA; NOVEMBER 1-8, 1999 ISSN: 1046-6673
- OKAJIMA KENJI: "Prevention of endothelial cell injury by activated protein C: the molecular mechanism(s) and therapeutic implications." CURRENT VASCULAR PHARMACOLOGY APR 2004, vol. 2, no. 2, April 2004 (2004-04), pages 125-133, XP001249017 ISSN: 1570-1611
- HASEGAWA NAOKI ET AL: "The effects of recombinant human thrombomodulin on endotoxin-induced multiple-system organ failure in rats" AMERICAN JOURNAL OF RESPIRATORY AND CRITICAL CARE MEDICINE, vol. 153, no. 6 PART 1, 1996, pages 1831-1837, XP009078174 ISSN: 1073-449X

## Description

### FIELD OF THE INVENTION

This invention relates to medical science particularly the prevention and treatment of acute renal failure with soluble thrombomodulin.

### BACKGROUND OF THE INVENTION

Hospital-acquired acute renal failure (ARF) continues to be associated with high mortality despite the technical advances in the care for these patients and improvements in our understanding of the pathophysiology of the disease process. Over the past 4 decades the mortality rate has remained constant and is in excess of 40-70%. This is especially true in the ICU setting where ARF is associated with a mortality of 50-90% (Conger JD. AmJKidDis 1995;26:565-76;.Liano F, Pascual J. Semin Nephrol 1998; 18:541-50; McCarthy JT, Mayo Clin Proc 1996;71(2): 17-26; Anderson RJ et al., N Engl J Med 1977;296(20):1134-8). Thus preventing ARF will improve mortality, and reduce morbidity, hospital length of stay and overall costs.

Numerous experimental models have shown that a variety of agents, including dopamine, osmotic agents, atrial natriuretic peptide, insulin like growth factor and endothelial receptor antagonists, while effective in animals, have been found to be ineffective in clinical studies of ARF (Solomon et al. N. Engl. J. Med. 1994;151:208-60; Allegren et al. N. EngI.J.Med. 1997;336:828-34; Hirschberg et al. Kidney Int. 999;55(6):2423-32; Brinkmann et al. J. Biol. Chem. 2002 14;277(24):21453-7).

Thus, there remains a need for therapeutics to treat ARF in humans.

Thrombomodulin (TM) is a glycoprotein present on the membrane surface of endothelial cells on many organs, including lung, liver, and kidney. Activated protein C (APC), is generated by thrombin-mediated cleavage of PC, an event which requires TM as a thrombin cofactor (Esmon, et al., J Biol Chem 257: 7944-7947, 1982; Esmon and Owen, Proc Natl Acad Sci USA 78: 2249-2252, 1981). When thrombin is complexed with TM in vivo, protein C (PC) activation is enhanced 1000-fold (Huang, et al., J Biol Chem 278: 46750-46759, 2003), and further enhanced 20 fold when PC is bound to endothelial cell PC receptor (ECPCR)(Esmon, Crit Care Med 32: S298-301, 2004). APC thus formed exerts anticoagulant effect by inactivating factors Va and VIIIa, thereby regulating the coagulation cascade. Recently it has been shown that APC protects against renal ischemic injury (Mizutani, et al., Blood 95: 3781-3787, 2000). Ischemic injury leads to release of many of cytokines that down regulate the expression of TM, hence causing a state of relative TM deficiency, and leaving the microvasculature in a pro-coagulant state (Ikeguchi, et al., Kidney Int 61: 490-501, 2002). It is assumed that this relative insufficiency of TM that occurs during and after ischemic injury due to hypoxia, stress, TNF-alpha and various other factors further worsening microvascular injury (Van de Wouwer and Conway, Crit Care Med 32: S254-261, 2004). Apart from its role in the PC system; TM has now been well established to possess roles in inflammation, fibrinolysis, apoptosis, cell adhesion and cellular proliferation (Conway, et al., J Exp Med 196: 565-577, 2002; Huang et al., J Biol Chem 278: 46750-46759, 2003). Thus using a soluble thrombomodulin offers a potentially significant approach to the prevention and treatment of ischemic ARF.

### SUMMARY OF THE INVENTION

The present invention provides a
1. soluble thrombomodulin for use in the treatment of a human subject having acute renal failure, wherein said soluble thrombomodulin is a soluble, secreted variant of thrombomodulin which lacks the full-length thrombomodulin transmembrane and cytoplasmic domains and optionally further lacks the ST domain.
2. Soluble thrombomodulin for use according to claim 1, wherein the acute renal failure results from inflammatory or ischemic injury.
3. Soluble thrombomodulin for use according to claim 1, wherein the acute renal failure is due to acute tubular necrosis resulting from renal ischemia.
4. Soluble thrombomodulin for use according to claim 1, wherein the acute renal failure is due to acute interstitial nephritis.

### DETAILED DESCRIPTION OF THE INVENTION

For purposes of the present invention, as disclosed and claimed herein, the following terms are as defined below.

ARF refers to Acute renal failure due to acute tubular necrosis or acute interstitial nephritis. ARF occurs when there is an acute reduction in glomerular filtration rate associated with the retention of nitrogenous wastes. Acute renal failure alternatively may be referred to as acute renal dysfunction.

APC refers to Activated protein C or aPC refers to recombinant aPC. APC includes and is preferably recombinant human aPC although aPC may also include other species having protein C proteolytic, amidolytic, esterolytic, and biological (anti-coagulant, anti-inflammatory, or pro-fibrinolytic) activities.

sTM refers to soluble thrombomodulin, which is a soluble, secreted variant of thrombomodulin which lacks the full-length thrombomodulin transmembrane and cytoplasmic domains. The primary amino acid structure of thrombomodulin is known in the art, as described in EP 0412841 A1. Human TM is synthesized as a 575 amino acid protein including a signal peptide portion reported to be 16, 18, or 21 residues in length. Following the signal peptide portion, human TM comprises the following domains or regions, sequentially from the amino terminus: 1) an amino terminal domain of -222-226 amino acids, 2) six EGF ("epidermal growth factor")-like structures of ∼236-240 amino acids, 3) a serine/threonine rich domain (ST domain) of∼34-37 amino acids and having several possible O-glycosylation sites, 4) a transmembrane region of -23-24 amino acids, and 5) a cytoplasmic domain of ∼36-38 amino acids. In the context of the present invention, sTM also includes a thrombomodulin derivative that further lacks the ST domain. Both forms of sTM possess thrombomodulin activity, as described below. As used herein, sTM is preferably recombinant sTM, and more preferably, human recombinant sTM.

Pharmaceutically effective amount refers to a therapeutically efficacious amount of a pharmaceutical compound. The particular dose of the compound administered according to this invention will, of course, be determined by the attending physician evaluating the particular circumstances surrounding the case, including the compound administered, the particular condition being treated, the patient characteristics and similar considerations.

Continuous infusion refers to continuing substantially uninterrupted the introduction of a solution or suspension into a vein for a specified period of time.

Bolus injection refers to the injection of a drug in a defined quantity (called a bolus) over a period of time up to about 120 minutes.

IRI refers to ischemia reperfusion injury.

Treating describes the management and care of a patient for the purpose of combating a disease, condition, or disorder whether to eliminate the disease, condition, or disorder, or prophylactically to prevent the onset of the symptoms or complications of the disease, condition, or disorder.

Thrombomodulin Activity refers to any property of soluble thrombomodulin or its derivatives responsible for protein C interaction, binding to thrombin, interaction with EPCR, having binding to HMGB1 and showing biological properties of APC cofactor activity, thrombin inhibitory activity and anti-inflammatory activity. Methods for testing for TM binding and functional activities are well known in the art, i.e., see Parkinson, et al., 1990 J. Biol. Chem. 265: 12602-12610; Grinnell and Berg. 1996. Am. J. Physiol. 270: H603-609; Gerlitz, et al 1993 Biochem. J. 295: 131-140; Abeyama et a]., 2005, J. Clin. Invest. 115:1267-1274.

PAC refers to partial aortic clamp.

The present invention provides for the treatment and/or prevention of acute renal failure with soluble thrombomodulin. Mortality from acute renal failure was previously thought to be related only to the underlying illness, recent studies indicate that renal failure per se is an independent risk factor for mortality (Levy EM et al JAMA 1996;275(19):1489-94; Chertow GM et al. Am J Med 1998;104(4):343-8). However, ARF that occurs secondary to another illness has a higher mortality and poorer long term outcome that ARF arising de novo (primary ARF).

A variety of agents in experimental models have been found effective in animals but ineffective in clinical studies (Solomon et al. N Engl J Med 1994; 151:208-60; Allegren et al. NEngIJMed 1997;336:828-34; Hirschberg et al. Kidney Int 999;55(6):2423-32; Brinkmann et al. J Biol Chem 2002 14;277(24):21453-7). Several factors contribute to these negative clinical results. First, human ARF is a complex disorder resulting from heterogeneous pathogenic factors. Therefore, targeting one selective pathophysiologic pathway with a selective agent is not likely to be beneficial. Previous failures also have arisen from the failure to apply treatment based upon our knowledge of the pathophysiology of acute kidney, injury, especially the role of inflammation. Furthermore, the therapeutic window appears to be narrow so that early initiation of therapy is imperative. Unfortunately, our present biological markers of ARF, serum creatinine and BUN (blood urea nitrogen), are poor surrogate markers of renal injury and dysfunction, including glomerular filtration rate (GFR). This scenario is compounded by the fact that an accurate GFR is extremely difficult to measure and interpret in unsteady state conditions. Furthermore, once changes in BUN and creatinine are detectable, significant ARF has already occurred and it may be beyond the therapeutic window.

Current strategies to minimize the occurrence of and limit the extent of injury of acute renal failure in high risk patients have been successful for certain causes of ARF. Most notably the incidence of radiocontrast nephropathy has been markedly reduced. However, when ARF occurs in pretreated high risk patients it still carries a five fold increase in mortality compared to matched patient populations (Levy EM et al JAMA 1996;275(19):1489-94). Furthermore, clinical scores incorporating risk factors to predict ARF will help in identifying patients who could potentially benefit from therapeutic agents given as a pretreatment (Tharkar CV et al J. Am. Soc. Nephrol 16:162-168, 2005). Our present understanding of the pathophysiology of ARF is based upon extensive studies identifying cellular and molecular mechanisms of acute renal failure (Bonventre JV and Weinberg JM J. Am. Soc. Neph 14:2199-2210,2003). Endothelial and epithelial cell injury and dysfunction are hallmarks of ARF. Recent data have now identified inflammation, especially endothelial-WBC interactions, as central issues to kidney damage during the Initiation and Extension phases of ARF(Molitoris and Sutton, Kidney Inter. 66:496-499, 2004.). Endothelial cell dysfunction and endothelial-inflammatory cells interactions including neutrophils, monocytes, macrophages and T cells have received considerable attention as important contributors to ischemic acute renal failure. Early inflammatory cell infiltration leads to further microvascular injury, inflammation, increased microvascular permeability, coagulation, apoptosis and necrosis. Early intervention to limit inflammation has recently received considerable interest as a protective strategy in preventing acute renal failure. Several new compounds appear to be effective in reducing injury for ischemia-reperfusion through direct action on leukocytes (Ortiz, et al., Transplant Proc 2003;35(4):1571-4; Day, et al., J Clin Invest 2003;112(6):883-91, Okusa, et al., Kidney Int 2001;59:2114-25; Cremer, et al. Ann Thorac Surg 1996;61(6):1714-20). Additional targets for therapeutic intervention include vasodilation, enhancing cellular repair and cellular differentiation. Although additional novel compounds have been proven effective in preclinical studies, many of these agents interrupt down stream targets that may not be effective in a complex disorder such as acute renal failure.

The complexity of acute kidney injury is in part due to the activation of multiple overlapping as well as distinct temporal pathways. Endothelial dysfunction and inflammation (cellular and humoral) are key mediators of ARF. It is unlikely that targeting events that occur late in the process of ARF will effectively reduce acute kidney injury. Therefore, new strategies to treat or prevent acute kidney injury will require compounds that target proximal pathways. Such strategies could include the use of compounds that broadly affect multiple pathways or combination therapies that target several areas rather than a single pathophysiologic focus.

A particularly illustrative example of ARF is following cardiac surgery. Two major processes contribute to ARF following cardiac surgery: 1) cardiopulmonary bypass (CPB) and 2) ischemia-reperfusion injury (IRI). Cardiopulmonary bypass (CPB) provokes a systemic inflammatory response syndrome (SIRS) (Faymonville, et al J Thorac Cardiovasc Surg 1991;102(2):309-17; Frering, et al., J Thorac Cardiovasc Surg 1994;108(4):636-41). Contact of blood components with the artificial surface of the bypass circuit, ischemia-reperfusion injury, endotoxemia, operative trauma, non-pulsatile blood flow and pre-existing left ventricular dysfunction are all possible causes of SIRS in this setting (Paparella, et al., Eur J Cardiothorac Surg 2002;21(2):232-44; Musial, et al., J Lab Clin Med 1985;105(4):514-22; Kirklin, et al., J Thorac Cardiovasc Surg 1983;86(6):845-57; Tennenberg et al., Ann Thorac Surg 1990;50(4):597-601). In its most severe form, a spectrum of injury may be observed that includes one or more of the following clinical manifestations: pulmonary, renal, gut, central nervous system, and myocardial dysfunction; coagulopathy; vasodilation and increased capillary permeability; hemolysis; pyrexia; and increased susceptibility to infection (Paparella, et al., Eur J Cardiothorac Surg 2002;21 (2):232-44.). During CPB, both neutrophils and vascular endothelium are activated (Asimakopoulos, et al., Ann Thorac Surg 1998;66(6):2135-44; Galinanes, et al., Circulation 1996;94(9Suppl):II364-9). Platelets also undergo activation, degranulation and adherence to vascular endothelium (Zilla et al., J Thorac Cardiovasc Surg 1989;97(3):379-88). These events lead to elaboration of cytotoxic oxygen-derived free radicals (Haga et al Artif Organs 1993;17(10):837-42), proteases (Faymonville et al., J Thorac Cardiovasc Surg 1991;102(2):309-17), cytokines (Frering et al., J Thorac Cardiovasc Surg 1994;108(4):636-41) and chemokines (Paparella et al., Eur J Cardiothorac Surg 2002;21(2):232-44). These inflammatory mediators, such as interleukin (IL)-6, IL-8 and tumor necrosis factor (TNF)-alpha, show a considerable rise in serum levels during CPB and in general reach peak levels 2 to 4 hours after termination of inflammation in CPB Ischemia-Reperfusion injury (IRI) is initiated by ischemia followed by pronounced activation of inflammatory cells, endothelial cells and epithelial cells. As a result, these target tissues (endothelial cells, circulating monocytes and tissue-fixed macrophages) release cytokines, and oxygen-derived free radicals that further drives the inflammatory response (Jansen et al., Ann Thorac Surg 1992;54(4):744-7; discussion 7-8). Despite efforts to produce a CPB system that does not produce contact activation of blood components, this goal has not been realized and CPB still remains a potent pro-inflammatory stimulus, often leading to ARF.

The present invention contemplates both the use of sTM or derivatives thereof for the prevention of acute renal failure in patients at high risk, as well as the treatment of acute renal failure resulting from inflammatory or ischemic injury. Patients at high risk include those with chronic kidney disease, underlying heart or liver disease, and diabetes who subsequently experience acute tubular necrosis or acute interstitial nephritis.

Soluble TM and its derivatives are useful for the prevention and treatment of acute tubular necrosis resulting from renal ischemia following major trauma or hemorrhage, cardiac arrest, cardiac bypass, septic shock, burns or any interrupted renal blood flow during surgery. In addition, soluble TM and derivatives will be useful following chemical injury from nephrotoxic drugs, thromboembolism, malignant hypertension, thrombotic thrombocytopenic purpura (TTP), hemolytic uremic syndrome (HUS), vasculitis, transfusion reaction, chemotherapy agents, toxins and poisons, radio contrast dyes used in imaging, malignant hypertension and disorders resulting from childbirth. Soluble TM and derivatives will also be useful for the treatment of acute interstitial nephritis caused by inflammation of interstitial kidney tissue following infection or in immune-related diseases such as lupus, leukemia, lymphoma, and sarcoidosis, and following kidney injury in response to antibiotics and NSAIDs (nonsteroidal anti-inflammatory drugs).

Methods to produce recombinant human soluble thrombomodulin have been described previously (Parkinson et al 1990 J. Biol. Chem. 265: 12602-12610; EP 0412841 A1).

The sTM is administered to a subject in need thereof using standard parenteral, peripheral administration techniques, with preferred routes of administration including intravenous and/or subcutaneous injection. More preferably, sTM will be administered either by IV bolus and/or subcutaneous injection using an appropriate dose for exposure ranging from one to twenty four or more hours, including but not limited to 48, 72, 96, or as many as 120 hours. The preparation of an acceptable pharmaceutical preparation of the sTM used in the present invention, including its strength, excipients, pH, isotonicity, presentation, dosage form, and the like, is well known to the skilled person.

Pharmaceutical compositions for use in the present invention should be appropriate for the selected mode of administration, and pharmaceutically acceptable excipients such as, buffers, surfactants, preservatives, solubilizing agents, isotonicity agents, stabilizing agents and the like are used as appropriate. Remington's Pharmaceutical Sciences, Mack Publishing Co., Easton PA, latest edition, provides a compendium of formulation techniques as are generally known to practitioners. Pharmaceutical preparations for use in the present invention should be sterile or at least nearly so, and if necessary preserved or rendered bacteriostatic.

The sTM will be used in conjunction with standard of care, including but not limited to appropriate antibiotic therapies to treat or prevent infection, diuretics for fluid management, furosemide or mannitol, dopamine, atrial natriuretic peptide (ANP), angiotensin-converting enzyme inhibitors, angiotensin receptor blockers, dialysis, erythropoietin, and treatment of hyperkalemia, acidosis, and potassium imbalance with Kayexalate, calcium, glucose/insulin, and/or diuretics.

The following examples are intended to illustrate but not to limit the invention. The examples describe experiments conducted in rats, thus the use of rat sTM is satisfactory. However, in the treatment methods of the invention intended for human use, recombinant, human sTM is preferred.

### Example 1

### Preparation and analysis of rat sTM

Methods to produce human recombinant soluble thrombomodulin have been described previously (Parkinson et al 1990 J. Biol. Chem. 265: 12602-12610.) For studies in rats, the analogous rat soluble thrombomodulin is produced as follows: Full length rat thrombomodulin is PCR amplified from a Clontech 5'- stretch plus cDNA rat lung library and TA cloned into pCRII-TOPO using the Invitrogen TOPO cloning kit using the following primers: 5' primer for full length rat TM,
5' - CGGTCTAGACCTGACCACCATGCTTGGGGTTTTCCTTCTGG - 3'; 3' primer for full length rat TM,
5'-GATGAGGTCGACGATATCTCA GAACTTCTGCAGCGTCCG - 3'. Following sequence confirmation for the full length rat TM cDNA, the following primers are used for PCR amplification and cloning rat soluble TM (sTM) into the mammalian transient expression vector XenoFLIS-PP-Fc, comprising the CMV promoter, preprotrypsin signal peptide, Xenopus leader and BGH poly-A:
5'primer for rat sTM, 5' - CCCAGGCTTCGACTAGCCAAGCTGCAGCCC - 3'; 3'primer for rat sTM, 5' - CCGCTCGAGTCAAGAGTGCACTGG CCTGGC - 3'. The rat sTM constructs do not include the ST domain as they are truncated 4 amino acids after EGF6. The resultant purified protein therefore has no chondroitin sulfate moiety (CS-).

Following sequence confirmation, the rat sTM expression vector is purified and used for a large scale transient expression in HEK293E cells. The conditioned media is concentrated and clarified by filtration. The conductivity of the concentrated, clarified conditioned medium is adjusted to 10 mS by addition of H₂O, prior to loading onto a Fast-flow Q-sepharose column (Amersham Biosciences), which has been equilibrated in buffer (20 mM Tris, pH 7.4, containing 50 mM NaCl, 5 mM EDTA, and 5 mM benzamidine-HCl). After loading, the column is washed with 3 bed-volumes of the same buffer, prior to elution with a 50 mM to 1 M NaCl linear gradient. Fractions containing thrombomodulin (by SDS-PAGE) are pooled, and the pH adjusted to 4.5, prior to dialysis in 20 mM sodium phosphate, pH 4.5, containing 5 mM EDTA and 5 mM benzamidine-HCl. The pooled protein is then clarified by centrifugation, and loaded onto an SP-sepharose column (Amersham Biosciences) equilibrated in 20 mM sodium phosphate, pH 4.55). Rat thrombomodulin, which is present in the column flow-through, is collected, concentrated, and further purified by gel-exclusion chromatography, using a Superdex S200 50/60 column (Amersham Biosciences) in PBS (10 mM sodium phosphate, pH 7.4, containing 150 mM NaCl). Fractions containing purified rat thrombomodulin were pooled, and sterile filtered using a 0.2 µM filter (Millipore). Protein concentration is determined by A₂₈₀, using an extinction coefficient of 1.1 (mg/mL)⁻¹cm⁻¹. MALDI mass spectrometry and N-terminal sequencing are used to confirm the identity and purity of the rat thrombomodulin. Endotoxin levels of rat sTM prepared in this manner were less than 5 EU/mg purified protein.

### Example 2

### Rodent partial aortic clamp model

Male Sprague-Dawley 200-250g rats are purchased from Harlan Laboratories (Indianapolis, IN). Rats are housed under standard laboratory conditions and fed a standard 10% corn oil-based rat chow and tap water ad libitum. The rats are allowed a minimum of 3 days acclimation period prior to starting the experimental protocols. The night before surgery, rats are denied access to food but have access to water. Anesthesia is induced with 5% halothane and maintained with 1-1.5% halothane in oxygen enriched air via a face mask. After shaving the abdomen of the rat, a midline incision is made through the skin and musculature to expose the abdominal cavity. The abdominal aorta just below the renal arteries is then isolated through blunt dissection from the inferior vena cava, and an ultrasonic probe (2.0mm diameter, Transit Time Perivascular Flowmeter TS420 (Transonic Systems, Inc, Ithica, NY) placed and secured to quantify the aortic blood flow rate. The upper abdominal aorta is then isolated through blunt dissection and freed from the surrounding structures to expose the aorta between the celiac artery and superior mesenteric artery (SMA).

The aortic clamp itself is comprised of two 4mm length polyethylene tubing (PE-100, 0.86mm diameter, Clay Adams Co, Parsippany, NJ) and a 10 inch 3.0 standard silk suture. The silk suture thread is first passed under the aorta in the above mentioned region. The first piece of tubing is then passed over both the ends of the thread to end up resting on the aorta between the celiac and SMA. The silk thread is then looped to leave an unsecured tie. The second piece of tubing is then placed in the loop, perpendicular and on top the first. The silk thread is then tied and the tension on the two ends of the thread increased until there is a 90% reduction of initial aortic blood flow rate as measured on the ultrasonic probe reader. This initial aortic blood flow rate is recorded prior to the placement of the tubings. Hence, a 10% baseline blood flow is maintained for a duration of 60 minutes. Rats are maintained on a warming blanket throughout the procedure to maintain body temperature of 37° C.

Once the surgery is complete, all rats in all experiments are given 2 ml of warm saline intraperitoneally to replace insensible and blood volume losses incurred during the surgery. The rats recover quickly from the anesthesia, and are monitored in warming-blanket provided cages for 4-6 hours post-operatively. Subsequently they are returned to their cages, allowed free access to food and water and cared for according to the guidelines of the Institutional Animal Care and Use Committee Review Board, who also approved of the above procedures.

Histopathological analysis is performed on a series of rats 24 hours after PAC. Prior to harvesting, kidneys are perfused briefly through the abdominal aorta with warm phosphate buffered saline (PBS) and subsequently preserved by *in vivo* perfusion with 4% paraformaldehyde (PFA) solution. Each rat has both kidneys harvested, cut into sagittal slices and immersed in PFA overnight at 4°C. The sections are then embedded in paraffin, and histologic staining with hematoxyline-eosin (H&E) or periodic acid-Schiff(PAS) is done. Histological grading for severity of tissue damage as assessed by extent of tubular cell sloughing, loss of proximal tubule brush border, cast formation, tubular dilatation and obstruction is performed by a renal pathologist (C.L.P.) blinded to the study,. Tubular necrosis scores as described previously (Jablonski, et al., Transplantation 35: 198-204, 1983) for cortical proximal tubule damage and (Kelly, et al J Clin . Invest 97: 1056-1063, 1996) for outer medulla tubular damage are also assessed,

*In vivo* two-photon microscopy is performed as previously described (Dunn, et al., Am J Physio Cell Physiol 283: C905-916, 2002; Sutton, et al., Kidney Int 62: 1539-1549, 2002). A total of 6 Sprague-Dawley rats undergo the PAC model and live renal imaging at 24h is performed using a Bio-Rad MRC-1024MP Laser Scanning Confocal/Multiphoton scanner (Hercules, CA) with an excitation wavelength of 800nm through a Nikon Diaphot inverted microscope utilizing a 60X NA 1.4 lens. A femoral venous catheter is inserted to gain vascular access for injecting dyes prior to imaging. Assessment of functional renal injury in the form of vascular permeability defects and disruptions in urinary and blood flow is achieved utilizing a nuclear stain(Hoechst-33342, 400ul, 1.5mg/ml in 0.9% saline; Molecular Probes, Eugene, OR), a high molecular weight dextran (HMWD) that is not filtered by the glomerulus under normal conditions(500,00ODa, 7.5mg/ml in 0.9% saline; Molecular Probes, Eugene, OR), and a low molecular weight dextran(LMWD), that is freely filterable (3,00ODa, 20mg/ml in 0.9% saline; Molecular Probes, Eugene, OR). To differentiate the two dextrans, the HMWD dextran is labeled with fluorescein (Molecular Probes, Eugene, OR) while the LMWD is labeled with Texas Red (Molecular Probes, Eugene, Oregon). The left kidney of the anesthetized rat is imaged following exteriorization through a retroperitoneal window via a flank incision. Images are analyzed with Metamorph (Universal Imaging, West Chester, PA) software. Approximately 10-12 images every 3 min are collected for each animal examined. For studies examining leukocytes in the microvasculature, images obtained are analyzed in a 4x4 grid. Leukocytes are identified by their characteristic to uptake the Hoechst nuclear stain. This is correlated with microvasculature and only leukocytes present in the microvasculature itself are counted. Leukocytes in the microvasculature are classified into 3 subtypes namely (i) free flowing - rapid appearance and disappearance during real time imaging in a grid for less than or equal to 2 frames, (ii) static or adherent - attached to microvascular endothelium with no movement, and (iii) rolling - appearance along endothelium surface for 3 or more frames in a grid.

All statistical analyses of plasma samples and daily weights utilize the two-sample, two-tailed unpaired Student's t-test of significance and linear regression where appropriate. All data in text and figures appear as the mean +/- two standard deviations of the mean. P-values are considered significant if less than 0.05 for all comparisons. Analysis is done using Microsoft Office Excel 8.0 Statistical software as well as EPIINFO v6.0 (CDC, Atlanta, GA).

### Example 3

### Effect of sTM following renal ischemia

A study is undertaken to develop a dose response curve for rat sTM serum levels and its antithrombotic efficacy. Recombinant rat sTM is administered to rats in a ferric chloride model (FeCl3) and time to occlusion is studied at various time points. The dose required to achieve a maximal antithrombotic effect is 5 mg/kg administered subcutaneously (s.c.). This response is maximal at 24 hours and persists through 48 hours post treatment but does not last through 72 hours. Simultaneous serum measurements of rat sTM using ELISA reveal that the maximum serum concentration achieved after a 5mg/kg subcutaneously is at 24 hours after administration. In a separate experiment, the intravenous pharmacokinetic data is established. The half life of sTM, 1mg/kg given intravenously (i.v.), is found to be approximately 4 hours. In all experiments the vehicle for sTM is saline and the volume injected is 0.76ml i.v, per rat and 1.64ml s.c. per rat.

The assessment of renal function and acid-base status is performed as follows: Blood samples in all cases are obtained from tail vein or aortic puncture at indicated time points after PAC under halothane. Serum creatinine,(SCr) is measured using Creatinine Analyzer 2(Beclanan-Coulter, Inc. Brea, CA) and is used to assess kidney function. Arterial blood gases (ABGs) and venous blood gases (VBGs) are obtained from their respective femoral catheters preoperatively at indicated time points and analyzed using blood gas analyzer ABL 77 (Radiometer Medicals, Copenhagen, Denmark).

It is determined by the initial studies that a partial suprarenal aortic clamp (PAC) at 90% intensity for a duration of 60 minutes yields a significant, but reversible renal failure as defined by a 3 to 8-fold increase in serum creatinine (SCr 0.75-2.5 mg/dL) at 24 hours with return to baseline levels over a 5 day period. A mortality rate of 20% is also observed at this intensity and duration. To evaluate the effect of pretreatment with sTM on ischemic ARF using the PAC model described above, rats are divided into two groups. One group (treated) is given 5mg/kg of sTM s.c. 24 hours prior to the surgery, while the other group (untreated)) is given an equal volume of saline 24h prior to surgery. All rats are followed and observed for 4 days post ischemic injury and renal function assessment using serum creatinine is done daily.

As shown in Table 1, untreated rats that underwent PAC I-R exhibit significant increases in serum creatinine. In comparison with untreated ischemic rats, the administration of a single s.c. dose of sTM (5mg/kg) given 24 hours before PAC ischemic injury produces a significant reduction in serum levels of creatinine in three different sets of experiments. This effect is significant at both 24 and 48 hours. Ischemic untreated rats experienced a mortality of 25%, similar to that seen during the prior experiment with PAC. However none of the sTM treated rats die. For the entire duration of the experiments, there is no incidence of more than expected bleeding during the surgery or bleeding during any procedure thereafter.

**Table 1 - Effect of sTM on renal function following ischemic injury**

| **Time (days)** | **sTM** | **Serum Creatinine** |
|---|---|---|
| 1 | - | 1.78 +/- 0.22 |
| | + | 0.52 +/- 0.27 p< 0.0001 |
| 2 | - | 1.43 +/- 0.32 |
| | + | 0.32 +/- 0.16 p< 0.005 |
| 3 | - | 0.3 +/- 0.14 |
| | + | 0.28 +/- 0.17 |
| 4 | - | 0.25 +/- 0.1 |
| | + | 0.18 +/- 0.05 |

Histological assessment and functional live 2-photon imaging at 24h is assessed in another group of rats who receive pretreatment with sTM 5mg/kg s.c. 24h prior to 60 min of ischemic injury using the PAC model and compared with ischemic untreated rats undergoing the same injury.

Untreated rats subjected to PAC I-R demonstrate significant medullary vascular congestion seen on gross morphology of the harvested kidneys at 24h. On histological examination a significant degree of renal injury is seen with extensive tubular dilatation, luminal congestion with casts, degeneration of tubular structure, necrosis, loss of brush border, and neutrophilia. The gross morphology of the sTM treated rats seen at 24h reveals decreased medullary vascular congestion. In contrast to untreated ischemic rats, renal sections obtained from rats treated with sTM (5mg/kg) given 24 hours prior to injury, demonstrate marked reduction in the severity of these histological features. Proximal tubule injury in the cortex is significantly less in the sTM pretreated group as compared to the untreated group. Similarly tubular damage in the outer medulla is significantly less severe in the sTM pretreated group (Table 2).

**Table 2. - sTM reduces kidney necrosis**

| | | **Necrosis Score** | |
|---|---|---|---|
| **Treatment** | | **Outer Medulla** | **Cortex** |
| sTM | | 0.8 +/- 0.447214 | 0.6+/-0.547723 |
| Control | | 3.2+/-0.447214 | 3.5+/-0.353553 |

### Example 4

### Renal Microvasculature

Soluble Thrombomodulin diminishes the increase in microvascular permeability following renal ischemia. Intravital 2-photon microscopy is used to examine changes in renal microvasculature permeability to investigate the effect of sTM on the integrity of microvasculature. The defect is shown to be most extensive at 24 hours following ischemia; hence this time point is chosen for imaging. In the saline treated control rats we observe leakage of both LMWD and the HMWD from the renal microvasculature. The extent of leakage of HMWD is less than that of LMWD. Following ischemia in sTM treated rats given 5mg/kg subcutaneous 24 hours prior to ischemia, the extent of LMWD is significantly less than that observed in saline treated control animals. Leakage of HMWD is virtually not seen in any sTM pretreated animals.

### Example 5

### Renal Ischemia

Soluble thrombomodulin decreases leukocyte adhesion following renal ischemia. Using intravital 2-photon microscopy the effect of ischemia on the dynamic nature of leukocyte adhesions and interactions that take place with the microvascular endothelium is studied. Under physiologic circumstances, all leukocytes are free flowing in the renal microvasculature. However 24 hours after PAC I-R injury, there is evidence of increased leukocyte adhesiveness to the endothelium both in terms of fully adherent or static leukocytes (12.9%) in the microvasculature as well as intermittent adhesions (rolling) with the endothelium (18.2%). Consequently the percentage of free flowing leukocytes is decreased in control ischemic animals (69.5%). In contrast, the sTM treated animals demonstrate a higher percentage of free flowing leukocytes (88.3%), and a significantly lower percentage of rolling (8.3%) or static leukocytes (3.3%). All these difference between the untreated and sTM treated rats are statistically significant (P<0.05).

sTM treated rats exhibit faster blood flow rates as compared to untreated rats which have turbulent, sluggish flow. In the untreated rats, casts inside tubular lumen (membrane blebs, cellular fragments) obstructed flow, tubular damage, and tubular necrosis is observed more frequently, whereas in the sTM treated group there are noticeably fewer luminal casts, better flow rate in most areas, and less tubular damage.

### Example 6

### Renal Function

To evaluate the effect on renal function of sTM given after ischemic injury, sTM is administered 2 hours following reperfusion. The dose of sTM administered is 1mg/kg i.v. along with a simultaneous dose of 5mg/kg s.c. The untreated ischemic rats are given similar volumes of saline through similar routes. The rationale to choose this dose and simultaneous i.v. and s.c. administration is based on the half-life of sTM as measured in separate experiments mentioned above, to allow sufficient serum levels for at least 24 hours post injury. The therapeutic time of 2 hours post-injury is chosen to mimic a human clinical scenario with a therapeutic realistic time window, when it is practical to employ an agent after a known ischemic insult has occurred. The rats are followed for 48h and assessment of renal function, acid-base status, hematocrits, muscle and liver enzymes are made.

Untreated rats that underwent PAC I-R exhibit a significant increase in serum creatinine. In comparison with controls, the simultaneous administration of sTM 1mg/kg i.v. and 5mg/kg s.c. 2 hours after reperfusion, significantly attenuates the renal dysfunction at 24 hours caused by PAC I-R (Table 3).

The mean serum creatinine in the sTM treated group is also lower than the untreated group at 48h, approaching statistical significance (P=0.08). In the 2 hour post injury treatment protocol, ischemic untreated rats experience a mortality rate of 45% at 24 hours, while none of the sTM treated rats die in the 2h post treatment protocol.

**Table 3 - Effect of sTM on renal function following ischemic injury with delayed treatment**

| **Time (days)** | **sTM** | **Serum Creatinine** |
|---|---|---|
| 0 | - | 0.31 +/- 0.022 |
| | + | 0.32+/-0.075 |
| 1 | - | 2.3 +/- 0.99 |
| | + | 0.68 +/- 0.44 p< 0.01 |
| 2 | - | 1.94 +/- 1.56 |
| | + | 0.81 +/- 0.76 |

Untreated ischemic rats that underwent PAC I-R have significant elevation in LDH, CK, AST and ALT suggesting significant ischemia-reperfusion injury to the muscles of the lower extremity, intestines and liver. Administration of sTM 1mg/kg i.v. along with simultaneous 5mg/kg s.c. attenuate this rise in enzymes significantly (Table 4). Because these enzymes markers are not specific for any organ and can be released from various sites undergoing ischemia-reperfusion injury, it is suffice to say that sTM attenuates the overall body injury induced by the partial aortic clamp ischemia reperfusion.

All rats have other serum chemistries and hematocrit measured. Both groups have similar declines in hematocrit at 24 and 48h as expected after I-R ARF. This finding is important as it supports the fact that there is no increase incidence of bleeding in the sTM treated group.

**Table 4 - Effect of sTM on markers of tissue injury**

| | **LDH*** | **CK** | **AST** | **ALT** |
|---|---|---|---|---|
| **Mean Control** | 1068.4 | 209.6. | 557-.8 | 97.6 |
| **SD Control** | 354.3195 | 23.13655 | 282.4973 | 53.67774 |
| **Mean sTM** | 393.8333 | 100 | 143.8333 | 38.66667 |
| **SD sTM** | 171.4916 | 51.93457 | 99.63416 | 9.770705 |
| **P-value** | 0.002493 | 0.001867 | 0.008166 | 0.02583 |

| | | | | |
|---|---|---|---|---|
| * LDH (Lactate dehydrogenase); CK (creatine kinase); AST (aspartate transaminase); ALT (alanine transaminase). | | | | |

## Claims

1. soluble thrombomodulin for use in the treatment of a human subject having acute renal failure, wherein said soluble thrombomodulin is a soluble, secreted variant of thrombomodulin which lacks the full-length thrombomodulin transmembrane and cytoplasmic domains and optionally further lacks the ST domain.

2. Soluble thrombomodulin for use according to claim 1, wherein the acute renal failure results from inflammatory or ischemic injury.

3. Soluble thrombomodulin for use according to claim 1, wherein the acute renal failure is due to acute tubular necrosis resulting from renal ischemia.

4. Soluble thrombomodulin for use according to claim 1, wherein the acute renal failure is due to acute interstitial nephritis.

## Patentansprüche

1. Lösliches Thrombomodulin zur Verwendung bei der Behandlung eines menschlichen Patienten mit akutem Nierenversagen, wobei das lösliche Thrombomodulin eine lösliche, sezernierte Variante von Thrombomodulin ist, der die die volle Länge aufweisenden Thrombomodulintransmembran- und -zytoplasmadomänen fehlen und der optional ferner die ST-Domäne fehlt.

2. Lösliches Thrombomodulin zur Verwendung nach Anspruch 1, wobei das akute Nierenversagen von einer entzündlichen oder ischämischen Verletzung herrührt.

3. Lösliches Thrombomodulin zur Verwendung nach Anspruch 1, wobei das akute Nierenversagen auf eine akute Tubulusnekrose, die von einer Nierenischämie herrührt, zurückzuführen ist.

4. Lösliches Thrombomodulin zur Verwendung nach Anspruch 1, wobei das akute Nierenversagen auf eine akute interstitielle Nephritis zurückzuführen ist.

## Revendications

1. Thrombomoduline soluble pour l'utilisation dans le traitement d'un sujet humain présentant une insuffisance rénale aiguë, dans laquelle ladite thrombomoduline soluble est une variante de la thrombomoduline soluble, sécrétée qui ne possède pas les domaines transmembranaires et cytoplasmiques de pleine longueur de la thrombomoduline et éventuellement qui ne possède en outre pas le domaine ST.

2. Thrombomoduline soluble pour l'utilisation selon la revendication 1, dans laquelle l'insuffisance rénale aiguë résulte de lésion inflammatoire ou ischémique.

3. Thrombomoduline soluble pour l'utilisation selon la revendication 1, dans laquelle l'insuffisance rénale aiguë est due à une nécrose tubulaire aiguë résultant d'une ischémie rénale.

4. Thrombomoduline soluble pour l'utilisation selon la revendication 1, dans laquelle l'insuffisance rénale aiguë est due à une néphrite interstitielle aiguë.
